# EUROPEAN PATENT SPECIFICATION

(11) **EP 3 295 902 B1**
(45) Date of publication and mention of the grant of the patent: **21.08.2019**
(21) Application number: 17190077.2
(22) Date of filing: 08.09.2017
(51) Int. Cl.: A61F 2/915, A61F 2/00, A61F 2/82, A61F 2/88

(54) **STENT**
STENT
ENDOPROTHÈSE

(30) Priority: 20.09.2016 JP 2016183251
(43) Date of publication of application: 21.03.2018
(73) Proprietor: Terumo Kabushiki Kaisha, Shibuya-ku Tokyo (JP)
(72) Inventor: KITO, Takayuki, Ashigarakami-gun, Kanagawa 259-0151 (JP)
(74) Representative: Casalonga

(56) References cited:
- EP-A1- 3 034 047
- WO-A1-2010/030928
- WO-A2-03/075797
- US-A1- 2014 364 935

## Description

### CROSS-REFERENCES TO RELATED APPLICATIONS

This application claims priority to Japanese Application No. 2016-183251 filed on September 20, 2016.

### TECHNICAL FIELD

The present disclosure relates to a stent.

### BACKGROUND ART

A stent is indwelled in a stenosed site or an occluded portion generated in a body lumen such as blood vessels or the like in an expanded state to maintain the body lumen in an opened state, and hence is required to have a strength for maintaining the expanded state. In addition, the stent is also required to have flexibility for following the shape of the body lumen. Therefore, various attempts have been made to improve flexibility.

For example, International Publication No. WO 2015/045101 discloses a stent having helical shaped struts connected with each other at link portions. The link portion includes connection sections disposed in an opposed state and a biodegradable material (bioabsorbable polymer) that connects the connection sections.

The helical shaped strut is higher in flexibility than a strut having an annular shape and desirably follows the shape of the body lumen. In addition, after the stent is indwelled in the body lumen, the biodegradable material is decomposed to release the connection between the connection sections after a predetermined period has elapsed. Accordingly, a higher flexibility can be demonstrated.

However, since the helical shaped strut has a high flexibility, the helical shaped strut can be easily deformed by an external force. The stent indwelled in the body lumen, which is curved or tortuous, may be subjected to a force in a bending direction or in a direction of compression. When the stent is subject to a force in the bending direction and the direction of compression after the connection between the connection sections is released, the struts may be deformed and the connection sections move in association with the deformation of the struts and thus may be overlapped with each other along a radial direction. If the connection sections are overlapped with each other along the radial direction, the thickness of the stent is increased at the overlapped portion, and thus the diameter of the stent is reduced. When the diameter of the stent is reduced, a smooth flow of blood flowing inside the stent may be hindered.

For example, it is considered that overlapping of the connection sections with each other along the radial direction may be prevented by adjusting the positions of the connection sections with respect to the direction of helix of the strut. However, distribution of the connection sections suitable for preventing overlapping of the connection sections with each other along the radial direction has not been sufficiently studied.

### SUMMARY OF DISCLOSURE

A stent is disclosed, according to claim 1, which is capable of preventing connection sections from overlapping with each other along the radial direction when a force is applied inadvertently thereto by adjusting distribution of the connection sections with respect to the direction of helix of the strut.

A stent is disclosed comprising: linear struts that define an outer periphery of a cylindrical shape with gaps therebetween; and link portions that connect the struts with the gaps, wherein the struts define a plurality of helical portions formed into a helical shape about an axis extending along an axial direction of the cylindrical shape, and wherein the link portion comprises a first connection section and a second connection section provided integrally with a first helical portion and a second helical portion adjacent to each other, and disposed at positions at least partly overlapping with each other along the axial direction of the cylindrical shape in a state of opposing each other, and a biodegradable material configured to connect the first connection section and the second connection section by being interposed between the first connection section and the second connection section. The first helical portion is disposed on a beginning end side of the plurality of helical portions with respect to the second helical portion. At least part of the first connection section is disposed on the beginning end side of the plurality of helical portions with respect to at least part of the second connection section. The first connection section includes a first protruding portion protruding toward the second connection section, and a first housing portion continuing to the first protruding portion. The second connection section includes a second protruding portion protruding toward the first connection section, and a second housing portion continuing to the second protruding portion. The first housing portion of the first connection section is disposed on the beginning end side of the plurality of helical portions with respect to the second housing portion of the second connection section.

According to the stent having the configuration described above, when the stent is subjected to a force in a bending direction or a direction of compression so that the helical portion is deformed, the connection sections move away from each other in association with the deformation of the plurality of helical portions. Therefore, the connection sections may be prevented from overlapping with each other along the radial direction.

### BRIEF DESCRIPTION OF THE DRAWINGS

FIG. 1 is a perspective view of a stent of an exemplary embodiment.
FIG. 2 is a deployed view of the stent of the exemplary embodiment deployed by cutting part of an outer periphery thereof linearly along an axial direction.
FIG. 3A is a drawing of a portion 3A surrounded by a double-dashed chain line in FIG. 2 in an enlarged scale.
FIG. 3B is an enlarged cross-sectional view taken along the line 3B-3B of FIG. 3A.
FIG. 4 is an explanatory drawing illustrating a distribution of connection sections at a link portion of the exemplary embodiment.
FIG. 5A is a drawing illustrating a stent indwelled in a body lumen.
FIG. 5B is a drawing illustrating a state in which the stent is deformed by receiving a force in a direction of compression.
FIG. 5C is a drawing illustrating a state in which the stent is deformed by receiving a force in a bending direction.
FIG. 6A is a drawing illustrating a portion 6A surrounded by a double-dashed chain line in FIG. 5A in an enlarged scale,
FIG. 6B is a drawing illustrating a portion 6B surrounded by a double-dashed chain line in FIG. 5B in an enlarged scale.
FIG. 6C is a drawing illustrating a portion 6C surrounded by a double-dashed chain line in FIG. 5C in an enlarged scale.
FIG. 7A is a schematic drawing for explaining a distribution of the connection sections with respect to a direction of helix of a helical portion of the embodiment.
FIG. 7B is a drawing for explaining a state of the helical portions after being deformed by receiving a force.
FIG. 8A is a schematic drawing for explaining a distribution of the connection sections with respect to the direction of helix of the helical portions according to a comparative example.
FIG. 8B is a drawing illustrating helical portions in a state after being deformed by receiving a force.
FIG. 9 is an enlarged view of a link portion according to a modification 1.
FIG. 10 is an enlarged view of a link portion according to a modification 2.

### DESCRIPTION OF EMBODIMENTS

Referring now to the attached drawings, embodiments of the present disclosure will be described below. Dimensional ratios of the drawings are exaggerated for illustrative purposes and are different from actual ratios.

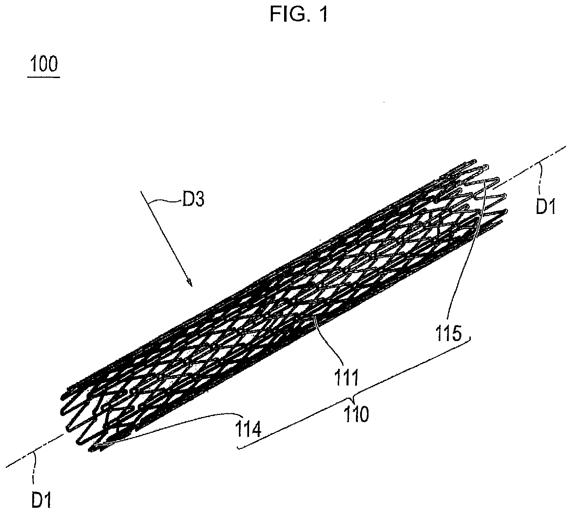

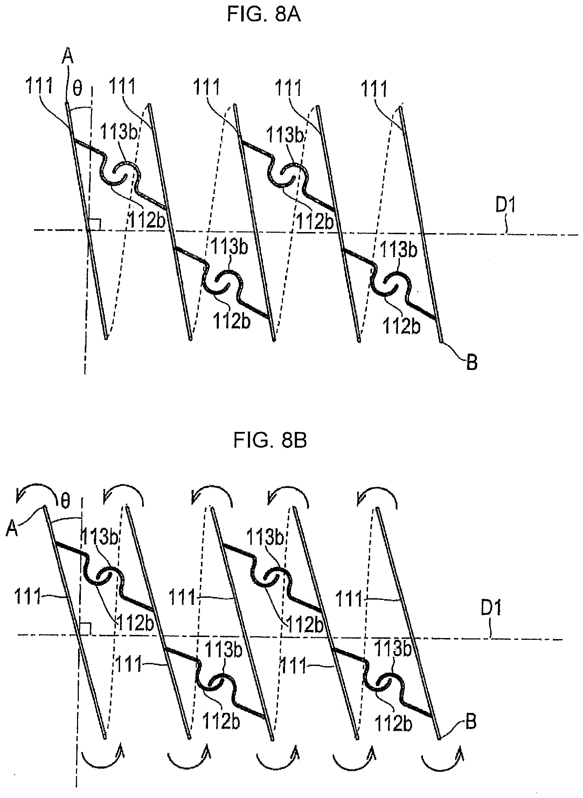

Referring now to FIG. 1 to FIG. 8B, a stent 100 of an exemplary embodiment will be described below.

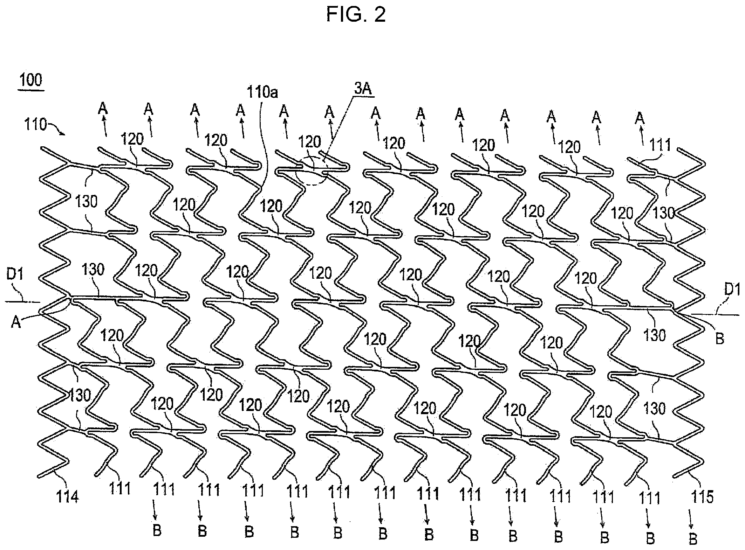

As illustrated in FIG. 1 and FIG. 2, the stent 100 of the embodiment includes a strut 110, which is a linear component, and a plurality of link portions 120, 130. The strut 110 defines an outer periphery of a cylindrical shape provided with gaps.

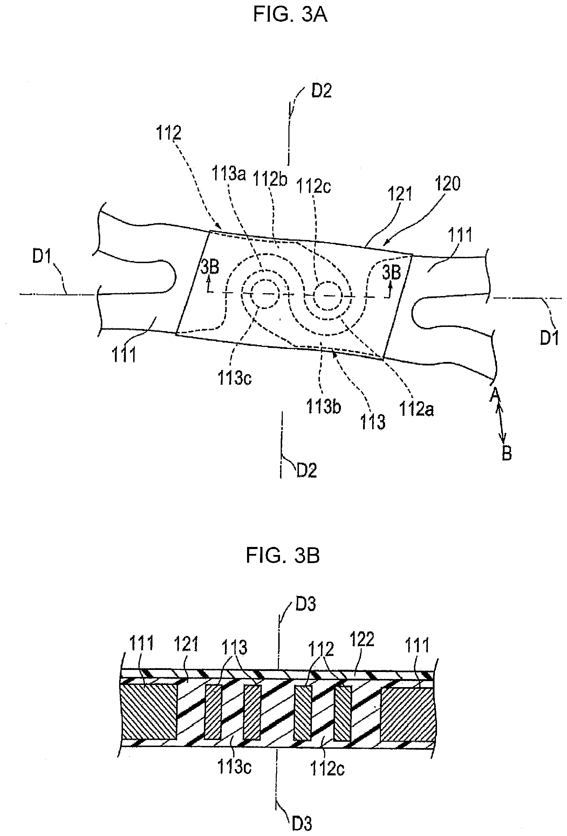

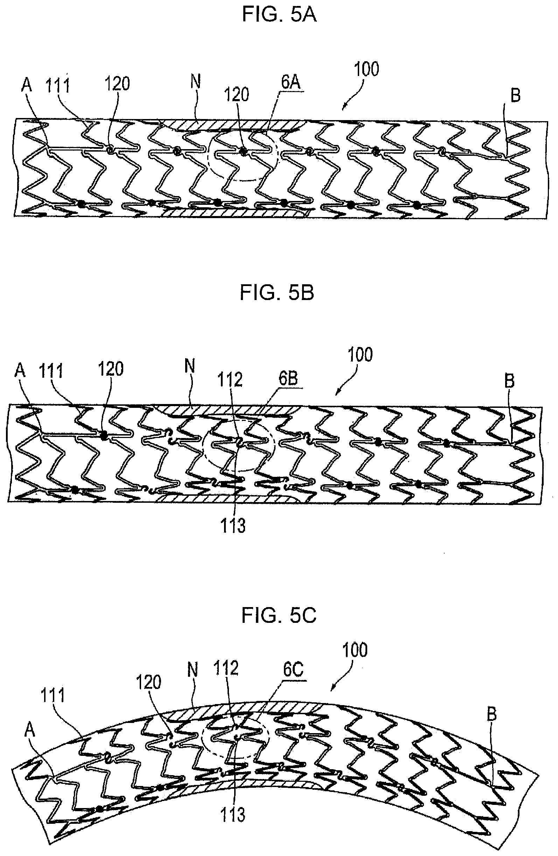

In the specification, an axial direction of the cylindrical shape formed by the strut 110 is referred to simply as an "axial direction D1" (see FIG. 1), an circumferential direction of the cylindrical shape is referred to simply as a "circumferential direction D2" (see FIG. 3A), and a radial direction of the cylindrical shape, that is, a thickness direction is referred to simply as a "thickness direction D3" (see FIG. 1 and FIG. 3B). In this disclosure, a side of being inserted into a living body (a right side in FIG. 5A) is referred to as a "distal side", and an operator side (the left side in FIG. 5A) is referred to as a "proximal side".

In accordance with an exemplary embodiment, the strut 110 defines annular portions 114, 115 located at both ends of the axial direction D1 and extending in the circumferential direction D2 to form an endless annular shape, and a plurality of helical portions 111 formed integrally into a helical shape about an axis extending along the axial direction D1 between the annular portion 114 and the annular portion 115.

The helical portion 111 is, as illustrated in FIG. 2, formed by helixes (a helix in a direction of a right screw) formed when the right screw advances while rotating from a beginning end A on the proximal side in the axial direction D1 connected to the annular portion 114 toward a terminal end B on the distal side in the axial direction D1 connected to the annular portion 115.

Note that in this disclosure, in a line extending along the helix of the helical portion 111, a side closer to the beginning end A on the proximal side in the axial direction D1 is referred to as a "beginning end A side", and a side closer to the terminal end B on the distal side in the axial direction D1 is referred to as a "terminal end B side". A direction along a helix in a direction of a right screw from the beginning end A side toward the terminal end B side is referred to as a "direction of a helix" of the helical portions 111.

The strut 110 includes a plurality of turned-back portions 110a bent by being turned back in a wavy shape.

In accordance with an exemplary embodiment, a material that forms the strut 110 is a non-degraded material, which is not degraded in a living body, for example. As the material described above, for example, stainless steel, cobalt based alloys such as a cobalt-chrome alloy (for example, CoCrWNi alloy), a resilient metal such as platinum-chrome alloys (for example, PtFeCrNi alloy), and a superelastic alloy such as a nickel-titanium alloy are exemplified.

The link portions 120 connect one helical portion (a first helical portion) 111 and an other helical portion (a second helical portion) 111 adjacent to each other in a gap between the one helical portion 111 and the other helical portion 111. Note that the one helical portion 111 is disposed on the beginning end A side with respect to the other helical portion 111.

The link portions 120 are disposed at regular intervals in a direction orthogonal to a direction of distance between the helical portions 111 adjacent to each other with a gap interposed therebetween.

As illustrated in FIG. 3A, the link portions 120 each include a first connection section 112, a second connection section 113, and a biodegradable material 121. The first connection section 112 is disposed on the beginning end A side with respect to the second connection section 113 (obliquely upper side on the left in FIG. 3A). In accordance with an exemplary embodiment, the first connection section 112 and the second connection section 113 are collectively referred to as "connection sections 112, 113".

The connection sections 112, 113 are integrally provided on the one helical portion 111 and the other helical portion 111 adjacent to each other respectively, and are connected by the biodegradable material 121 in a state of being opposed to each other.

The first connection section 112 is formed by the one helical portion 111 by partly protruding and the second connection section 113 is formed by the other helical portion 111 by partly protruding.

As illustrated in FIGS. 3A and 3B, the first connection section 112 includes a first protruding portion 112a protruding toward the second connection section 113 side and having a rounded curved shape, a first housing portion 112b continuing to the first protruding portion 112a and having a depressed shape in accordance with an outer shape of a second protruding portion 113a of the second connection section 113, and a first holding portion 112c formed so as to penetrate from a surface of the helical portion 111 in the thickness direction D3 for holding the biodegradable material 121. The second connection section 113 includes the second protruding portion 113a protruding toward the first connection section 112 side and having a rounded curved shape, a second housing portion 113b continuing to the second protruding portion 113a and having a depressed shape in accordance with an outer shape of the first protruding portion 112a of the first connection section 112, and a second holding portion 113c formed so as to penetrate from the surface of the helical portion 111 in the thickness direction D3 for holding the biodegradable material 121.

As illustrated in FIG. 3A, in a state in which the connection sections 112, 113 are connected by the biodegradable material 121, the first housing portion 112b of the first connection section 112 is disposed on the beginning end A side with respect to the second housing portion 113b of the second connection section 113.

In accordance with an exemplary embodiment, the depressed shape of the first housing portion 112b is formed so as to be larger than the outer shape of the second protruding portion 113a. The depressed shape of the second housing portion 113b can be formed so as to be larger than the outer shape of the first protruding portion 112a.

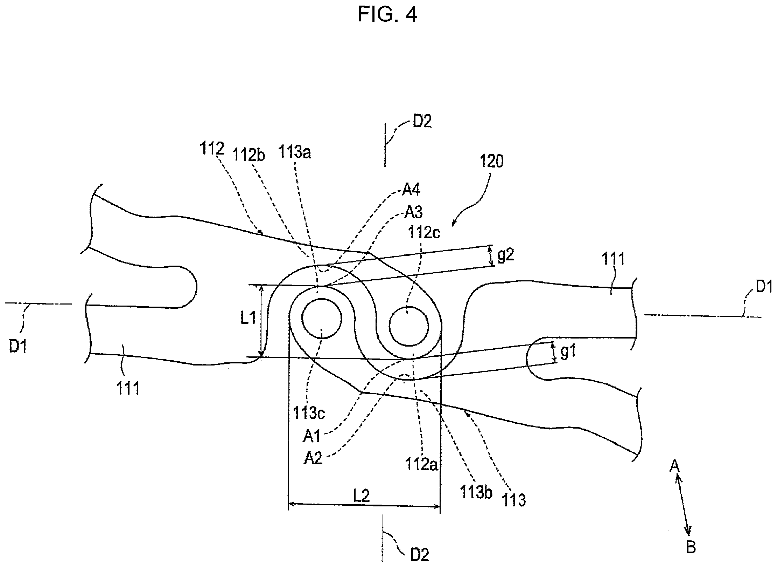

As illustrated in FIG. 4, the first protruding portion 112a is configured so as to be capable of being housed in the depressed shape of the second housing portion 113b. In a state in which the first protruding portion 112a is housed in the second housing portion 113b, a surface A1 of the first protruding portion 112a opposing the second housing portion 113b is disposed with a gap g1 from a surface A2 of the second housing portion 113b opposing the first protruding portion 112a.

The second protruding portion 113a is configured so as to be capable of being housed in the depressed shape of the first housing portion 112b. In a state in which the second protruding portion 113a is housed in the first housing portion 112b, a surface A3 of the second protruding portion 113a opposing the first housing portion 112b is disposed with a gap g2 from a surface A4 of the first housing portion 112b opposing the second protruding portion 113a.

Note that the first protruding portion 112a may be partly in contact with the second housing portion 113b. Note that the second protruding portion 113a may be partly in contact with the first housing portion 112b.

The connection sections 112, 113 in a state of being connected by the biodegradable material 121 are disposed at positions overlapped at least partly with each other along the axial direction D1 and the circumferential direction D2. In accordance with an exemplary embodiment, a length of the connection sections 112, 113 overlapped with each other along the circumferential direction D2 is L1. A length of the connection sections 112, 113 overlapped with each other along the axial direction D1 is L2.

The first holding portion 112c is disposed at a position overlapped with the second connection section 113 along the axial direction D1. The second holding portion 113c is disposed at a position overlapped with the first connection section 112 along the axial direction D1. As illustrated in FIG. 3B, in this exemplary embodiment, the respective holding portions 112c, 113c are formed of through holes penetrating in the thickness direction D3 of the helical portions 111. However, the respective holding portions 112c, 113c do not have to be the through holes as long as they can hold the biodegradable material 121, and may have a form depressed in the thickness direction D3 of the helical portions 111 at least to some extent.

As illustrated in FIGS. 3A and 3B, the biodegradable material 121 combines the first connection section 112 and the second connection section 113 from a time point when the stent 100 is indwelled in the body lumen for a predetermined time until the biodegradable material 121 is degraded.

The biodegradable material 121 is provided so as to integrally continue the surfaces of the connection sections 112, 113, the gaps between the first connection sections 112 and the second connection sections 113, and in the respective holding portions 112c, 113c. Not only providing the biodegradable material 121 so as to cover the surfaces of the connection sections 112, 113, but also combining the connection sections 112, 113 desirably by filling the interiors of the gaps between the first connection sections 112 and the second connection sections 113 and the interiors of the respective holding portions 112c, 113c with the biodegradable material 121.

In accordance with an exemplary embodiment, the biodegradable material 121 is not specifically limited as long as it is a material which is degraded in the living body, and such materials include, for example, biodegradable synthetic polymeric materials such as polylactic acid, polyglycolic acid, lactic acid-glycolic acid copolymer, polycaprolactone, lactic acid-caprolactone copolymer, glycolic acid-caprolactone copolymer, poly-γ-glutamic acid, biodegradable natural polymer materials such as collagen, and biodegradable metallic materials such as magnesium and zinc are exemplified.

As illustrated in FIG. 3B, the stent 100 including the link portions 120 are provided with a covering member 122 containing a drug on the surface of the stent 100. The covering member 122 is preferably formed on the surface of the biodegradable material 121, preferably, on the outer surface on the side opposing the inner peripheral surface of the body lumen. However, the disclosure is not limited thereto.

In accordance with an exemplary embodiment, the covering member 122 includes a drug capable of restricting proliferation of the neointima and a drug carrier for carrying the drug. Note that the covering member 122 may only be composed of the drug. The drug contained in the covering member 122 is at least one selected from a group including, for example, sirolimus, everolimus, zotarolimus, and paclitaxel. Although the components of the drug carrier are not specifically limited, biodegradable material is preferable and materials similar to the biodegradable material 121 may be applied.

The link portions 130 are integrally formed with the annular portions 114, 115 and the helical portions 111.

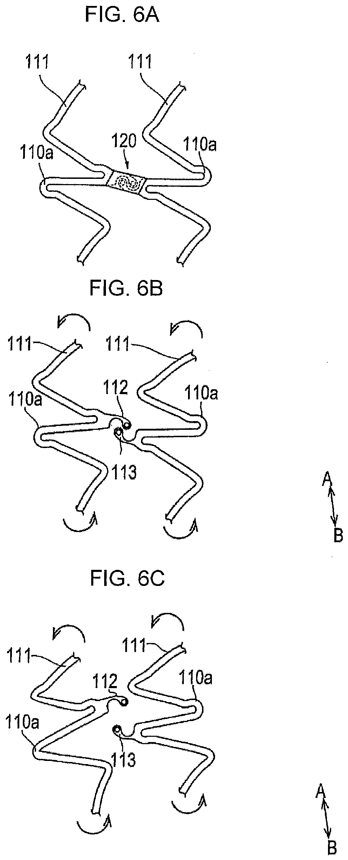

Referring now to FIGS. 5A to 5C and FIGS. 6A to 6C, advantageous effects of the stent 100 of this embodiment will be described. FIGS. 5A to 5C are drawings illustrating the stent 100 indwelled in the body lumen, FIGS. 6A to 6C are drawings illustrating the rink portions 120 of the stents 100 illustrated in FIGS. 5A to 5C, respectively, in an enlarged scale.

In accordance with an exemplary embodiment, the stent 100 is delivered to a stenosed site generated in the body lumen such as a blood vessel, a bile duct, a trachea, an esophagus or a urethra using a medical device for stent delivery such as a balloon catheter. The delivered stent 100 is being expanded and indwelled in a stenosed site N of the body lumen.

In the stent 100 of this exemplary embodiment, degrading of the biodegradable material 121 is not advanced so much shortly after the indwelling and the stent 100 is exposed from the inner surface of the body lumen, and as illustrated in FIG. 5A and FIG. 6A, connection between the link portions 120 is desirably maintained by the connection sections 112, 113 (see FIG. 3A). Therefore, as the stent 100 has a high strength and may maintain a significantly expanded state immediately after the indwelling with reliability, for example, catheters for IVUS (intravascular ultrasound examination) or catheters for OFDI (optical coherent tomography) or balloon catheters for posterior dilation applied for checking the indwelling state can be passed through inside of the stent 100.

As the stent 100 maintains a high strength, even when the illustrated respective devices described above unintentionally come into contact with the stent 100 passes therein, a risk of deformation of the stent 100 in the axial direction D1 (deformation) can be reduced.

Once the stent 100 endothelialization advances are covered by endothelium, the link portions 120 release the connection by the degradation of the biodegradable material 121.

By the release of the connection by the biodegradable material 121 at the link portions 120, the stent 100 can demonstrate a significantly high flexibility, and follows the shape of the body lumen flexibly. Consequently, the stent 100 is capable of supporting the body lumen with minimally invasive manner so as to keep the body lumen opened for a long time.

As illustrated in FIG. 5B, the stent 100 indwelled in the stenosed site N receives a force in the direction of compression (inward of the radial direction D3). When the force in the direction of compression is applied to the stent 100, as illustrated in FIG. 6B, the helical portions 111 are deformed so as to be inclined. Accordingly, the connection sections 112, 113 move in the direction away from each other. As used herein the term "in the direction away from each other" is intended to include a direction in which the gap g1 between the first protruding portion 112a and the second housing portion 113b and the gap g2 between the second protruding portion 113a and the first housing portion 112b are widened (see FIG. 4).

The stent 100 that has deformed by following the shape of the curved or tortuous shape of the body lumen may receive a force in a bending direction. When the stent 100 receives a force further in the bending direction from a state of having received a force in the direction of compression illustrated in FIG. 5B, the helical portions 111 are deformed so as to be further inclined as illustrated in FIG. 5C and FIG. 6C. Accordingly, the connection sections 112, 113 move further in the direction away from each other.

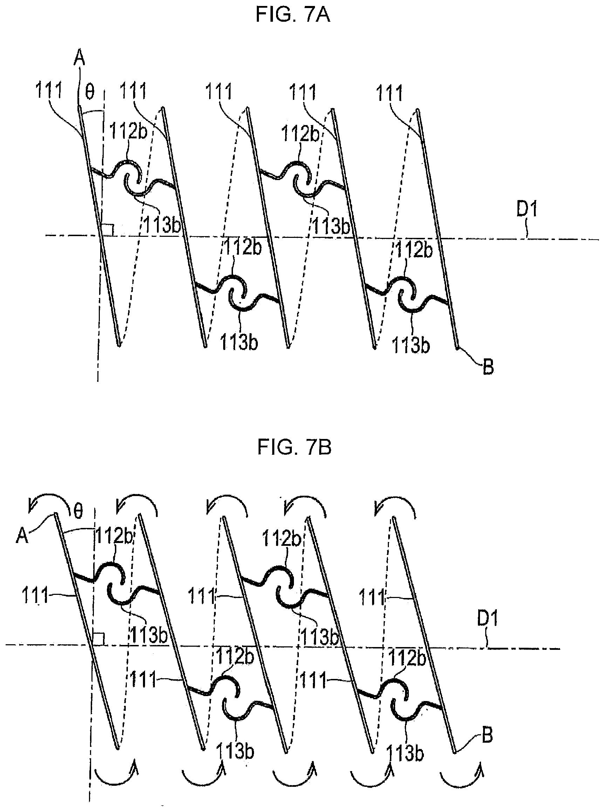

Referring now to FIGS. 7A, 7B, 8A, and 8B, a principle of movement of the connection sections 112, 113 in the direction away from each other when a force is applied to the stent 100 in the direction of compression and the bending direction will be described below. FIGS. 7A and 7B are schematic drawings for explaining distribution of the connection sections 112, 113 (the housing portions 112b, 113b) with respect to the direction of helix of the helical portions 111, and FIGS. 8A and 8B are schematic drawings for explaining the distribution of the connection portions (accommodating portions) with respect to the direction of the helix of the helical portion in a comparative example.

In the link portions 120 of the stent 100 according to the embodiment, the first connection sections 112 are disposed on the beginning end A side with respect to the second connection sections 113. The first housing portions 112b of the first connection sections 112 are disposed on the beginning end A side with respect to the second housing portions 113b of the second connection sections 113 (see FIG. 4). FIG. 7A illustrates this state schematically.

In accordance with an exemplary embodiment, when a force is applied to the stent 100 in the bending direction or in the direction of compression, the helical portions 111 are deformed so as to be inclined in such a manner that the angle of inclination θ with respect to a vertical axis which is orthogonal to the axial direction D1 is slightly increased as illustrated in FIG. 7B.

In accordance with the deformation of the helical portions 111, the first housing portion 112b of the connection sections 112 to be connected to the helical portions 111 of the beginning end A side moves to the beginning end A side, the housing second portion 113b of the connection sections 113 to be connected to the helical portions 111 of the terminal end B side moves to the terminal end B side. Accordingly, the connection sections 112, 113 move in the direction away from each other.

As illustrated in FIG. 8A as a comparative example, a case where the first connection sections 112 are disposed on the beginning end A side with respect to the second connection sections 113, and the first housing portion 112b of the first connection sections 112 is disposed on the terminal end B side with respect to the second housing portion 113b of the second connection sections 113 will be described. In this case, as illustrated in FIG. 8B, in accordance with the deformation of the helical portions 111, the first housing portion 112b of the connection sections 112 to be connected to the helical portions 111 of the beginning end A side moves to the beginning end A side, the second housing portion 113b of the connection sections 113 to be connected to the helical portions 111 moves to the terminal end B side. At this time, since the housing portions 112b, 113b are disposed at positions that hinders the movement of the connection sections 112, 113 with each other, the protruding portions 112a, 113a and the housing portions 112b, 113b move toward each other, and thus the connection sections 112, 113 overlap with each other along the radial direction D3. If the connection sections 112, 113 are overlapped with each other along the radial direction D3, the thickness of the stent 100 is increased at the overlapped portion, and thus the diameter of the stent 100 is reduced. In accordance with an exemplary embodiment, when the diameter of the stent 100 is reduced, smooth blood flow can be hindered, which may cause thrombus or the like.

As described above, according to the stent 100 of the embodiment, the link portions 120 includes the first connection sections 112 and the second connection sections 113 provided integrally with the one helical portions 111 and the other helical portions 111 adjacent to each other and disposed in the positions overlapped with each other at least partly along the axial direction D1 of the cylindrical shape in a state opposing to each other, and a biodegradable material 121 that connects the first connection sections 112 and the second connection sections 113 by being interposed between the first connection sections 112 and the second connection sections 113. The one helical portion 111 is disposed on the beginning end A side of the helical portions 111 with respect to the other helical portions 111. At least parts of the first connection sections 112 are disposed on the beginning end A side of the helical portions 111 with respect to at least parts of the second connection sections 113.

According to the stent 100 having the configuration described above, since at least parts of the connection sections 112, 113 are disposed at positions overlapped with each other along the axial direction D1 in a state of being connected by the biodegradable material 121, the state in which the connection sections 112, 113 are connected with each other may be maintained desirably until the biodegradable material 121 is degraded. After the biodegradable material 121 is degraded and thus the connection between the connection sections 112, 113 is released, if the stent 100 receives a force in the bending direction and the direction of compression, the helical portions 111 is deformed so that the angle of inclination θ with respect to the vertical axis which is orthogonal to the axial direction D1 is slightly increased. The connection sections 112, 113 move in the direction apart from each other in association with the deformation of the helical portions 111. Therefore, the connection sections 112, 113 may be prevented from being overlapped with each other along the radial direction D3. Accordingly, the stent 100 can be prevented from unintentionally being reduced in diameter after the stent 100 has been indwelled.

In accordance with an exemplary embodiment, the first connection sections 112 each include the first protruding portion 112a projecting toward the second connection section 113 side, and the second housing portion 112b that is continued to the first protruding portion 112a and having a depressed shape in accordance with the outer shape of the second protruding portion 113a, the second connection sections 113 each include the second protruding portion 113a projecting toward the connection section 112 side and the second housing portion 113b that is continued to the second protruding portion 113a and having the depressed shape in accordance with the outer shape of the first protruding portion 112a, and the first housing portion 112b of the first connection sections 112 is disposed on the beginning end A side of the helical portions 111 with respect to the second housing portion 113b of the second connection sections 113. Therefore, the connection sections 112, 113 may be prevented further easily from being overlapped with each other along the radial direction D3.

In a state in which the connection sections 112, 113 are connected by the biodegradable material 121, the first protruding portions 112a are housed in the second housing portions 113b and the second protruding portions 113a are housed in the first housing portions 112b, and one of the protruding portions 112a, 113a and the other one of the housing portions 112b, 113b are disposed at positions overlapped with each other along the circumferential direction D2. Therefore, from a moment when the stent 100 is indwelled in the body lumen and a predetermined period has elapsed until the biodegradable material 121 is degraded, the state in which the connection sections 112, 113 are connected may be desirably maintained.

Also, the first connection sections 112 and the second connection sections 113 respectively have the holding portions 112c, 113c formed so as to penetrate or be depressed from the surface of the strut 110 in the thickness direction D3 to hold the biodegradable material 121. The first holding portions 112c of the first connection sections 112 are disposed at positions overlapping with the second connection sections 113 along the axial direction D1. The second holding portions 113c of the second connection sections 113 are disposed at positions overlapping with the first connection sections 112 along the axial direction D1. The connection sections 112, 113 are each disposed so as to overlap with the other connection portions along the axial direction D1 to positions where the holding portions 112c, 113c are formed so that, the lengths of portions where the connection sections 112, 113 overlap with each other along the axial direction D1 may be set to be relatively long. Consequently, even when a force is inadvertently applied to the stent 100, the link portions 120 may maintain mechanical connection desirably. Therefore, from a moment when the stent 100 is indwelled in the body lumen and a predetermined period has elapsed until the biodegradable material 121 is degraded, the connection between the struts 110 may be desirably maintained.

In addition, the stent 100 includes the covering member 122 and drug, which may prevent proliferation of the neointima, is liquated gradually from the covering member 122, so that reoccurrence of stenosis at the stenosed site is further restricted.

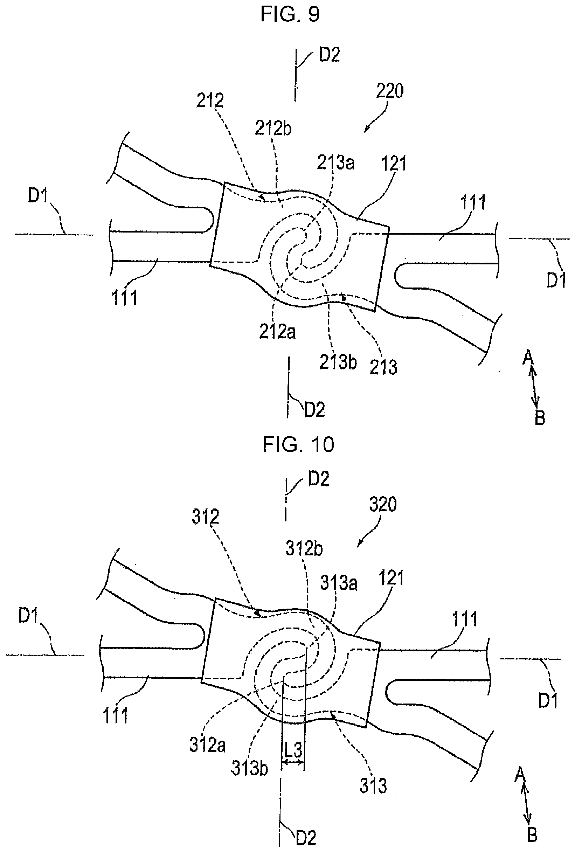

FIG. 9 is a drawing of a link portion 220 according to a modification 1. Note that portions having the same configurations as this embodiment are denoted by the same reference numerals and description is omitted.

The link portions 220 according to Modification 1 are different from the link portions 120 of the embodiment described above in that connection sections 212, 213 are not provided with a holding portion. The link portions 220 of Modification 1 will be described in detail below.

The link portions 220 include the first connection section 212, the second connection section 213, and a biodegradable material 121 as illustrated in FIG. 9. The first connection section 212 and the second connection section 213 are integrally provided on the one helical portion 111 and the other helical portion 111 adjacent to each other respectively, and is connected by the biodegradable material 121 in a state of being opposed to each other.

The first connection sections 212 each include a first protruding portion 212a protruding toward the second connection section 213 side, and a first housing portion 212b that is continued to the first protruding portion 212a and having a depressed shape in accordance with the outer shape of a second protruding portion 213a of the second connection section 213. The second connection section 213 each include the second protruding portion 213a protruding toward the first connection section 212 side, and a second housing portion 213b that is continued to the second protruding portion 213a and having a depressed shape in accordance with the outer shape of the first protruding portion 212a of the first connection section 212.

The first protruding portion 212a and the first housing portion 212b are formed integrally by a curved linear member so as to draw a semi-circle. In the same manner, the second protruding portion 213a and the second housing portion 213b are formed integrally by a curved linear member so as to draw a semi-circle. Accordingly, after the biodegradable material 121 is degraded and the connection between the connection sections 212, 213 is released, even though a force is applied unintentionally to the stent 100, the connection sections 212, 213 move in the direction away from each other without interfering with each other. Therefore, the connection sections 212, 213 may be prevented from overlapping with each other along the radial direction D3.

FIG. 10 is a drawing of a link portion 320 according to a modification 2. Note that portions having the same configurations as this embodiment are denoted by the same reference numerals and description is omitted.

In accordance with an exemplary embodiment, the link portions 320 of Modification 2 are different from the link portions 220 of Modification 1 described above in that at least parts of one protruding portion 312a and the other protruding portion 313a are disposed at positions overlapping each other along the axial direction D1. The link portions 320 of Modification 2 will be described in detail below.

The link portions 320 include a first connection section 312, a second connection section 313, and a biodegradable material 121 as illustrated in FIG. 10. The first connection section 312 and the second connection section 313 are integrally provided on one helical portion (a first helical portion) 111 and an other helical portion 111 (a second helical portion) adjacent to each other respectively and are connected by the biodegradable material 121 in a state of being opposed to each other.

The first connection section 312 each include the first protruding portion 312a protruding toward the second connection section 313 side, and a first housing portion 312b that is continued to the first protruding portion 312a and having a depressed shape in accordance with the outer shape of a second protruding portion 313a of the second connection section 313. The second connection section 313 each include the second protruding portion 313a protruding toward the first connection section 312 side, and a second housing portion 313b that is continued to the second protruding portion 313a and having a depressed shape in accordance with the outer shape of the first protruding portion 312a of the first connection section 312.

The protruding portions 312a, 313a in a state of being connected by the biodegradable material 121 are disposed at positions overlapped at least partly with each other along the axial direction D1. A length of the protruding portions 312a, 313a overlapped with each other along the axial direction D1 is L3.

Therefore, with the protruding portions 312a, 313a disposed so as to overlap with each other along the axial direction D1, the state in which the connection sections 312, 313 are connected may be maintained desirably from a moment when the stent 100 is indwelled in the body lumen and a predetermined period has elapsed until the biodegradable material 121 is degraded.

The disclosure is not limited to the above-described embodiment and modifications, and includes other various modifications within a scope of claims.

For example, only one link portion out of the plurality of link potions is required to have the first connection section, the second connection section, and the biodegradable material, and the type of the link portions is not limited to the embodiment and the modifications described above. For example, in the embodiment and the modifications described above, the link portions 130 may be composed of the first connection sections 112, 212, 312, the second connection sections 113, 213, 313, and the biodegradable material 121 in the same manner as the link portions 120.

In addition, the helical portion 111 may be formed by helixes (a helix in a direction of a left screw) formed when the left screw advances while rotating from the beginning end A on the proximal side in the axial direction D1 connected to the annular portion 114 and the terminal end B on the distal end side in the axial direction D1 connected to the annular portion 115.

The distribution of the link portions is not limited to the embodiment and the modifications described above, and may be modified as needed.

The mode of the struts is not limited to the embodiment and the modifications described above. The helical portions of the stent of the present disclosure may be formed of a helix, which does not include the turned-back portions 110a such as in the embodiment and the modifications described above.

The outer shapes of the protruding portion, the housing portion, and the holding portion are not limited to the embodiment and modifications described above. For example, the outer shapes of the protruding portion, the housing portion, and the holding portion may be formed into a given polygonal shape.

In accordance with an exemplary embodiment, the strut 110 of the embodiment described above is formed of a non-biodegradable material. However, the present disclosure is not specifically limited thereto. For example, in accordance with an exemplary embodiment, the strut may be formed of a biodegradable material, which is degraded slower than the biodegradable material included in the link portions.

Also the present disclosure includes a mode including no covering member 122, and a mode including a drug, which is capable of preventing proliferation of the neointima in the biodegradable material 121. In the latter mode, the drug is gradually eluted in association with degradation of the biodegradable material 121, so that reoccurrence of stenosis at the stenosed site is restricted.

The detailed description above describes a stent.

## Claims

1. A stent (100) comprising:
linear struts (110) that define an outer periphery of a cylindrical shape with gaps therebetween; and
link portions (120, 130, 220, 320) that connect the struts (110) with the gaps, wherein the struts (110) define a plurality of helical portions (111) formed into a helical shape about an axis extending along an axial direction (D1) of the cylindrical shape, and wherein the link portion (120, 130, 220, 320) comprises a first connection section (112, 212, 312) and a second connection section (113, 213, 313) provided integrally with a first helical portion (111) and a second helical portion (111) adjacent to each other, and disposed at positions at least partly overlapping with each other along the axial direction (D1) of the cylindrical shape in a state of opposing each other, and a biodegradable material (121) configured to connect the first connection section (112, 212, 312) and the second connection section (113, 213, 313) by being interposed between the first one connection section (112, 212, 312) and the second connection section (113, 213, 313), wherein
the first helical portion (111) is disposed on a beginning end side (A) of the plurality of helical portions (111) with respect to the second helical portion (111), and
at least part of the first connection section (112, 212, 312) is disposed on the beginning end side (A) of the plurality of helical portions (111) with respect to at least part of the second connection section (113, 213, 313), wherein
the first connection section (112, 212, 312) includes:
a first protruding portion (112a, 212a, 312a) protruding toward the second connection section (113, 213, 313), and
a first housing portion (112b, 212b, 312b) continuing to the first protruding portion (112a, 212a, 312a) and,
the second connection section (113, 213, 313) includes:
a second protruding portion (113a, 213a, 313a) protruding toward the first connection section (112, 212, 312), and
a second housing portion (113b, 213b, 313b) continuing to the second protruding portion (113a, 213a, 313a),
**characterized in that**
the first housing portion (112b, 212b, 312b) of the first connection section (112, 212, 312) is disposed on the beginning end side (A) of the plurality of helical portions (111) with respect to the second housing portion (113b, 213b, 313b) of the second connection section (113, 213, 313).

2. The stent (100) according to Claim 1, wherein the first housing portion (112b, 212b, 312b) has a depressed shape in accordance with an outer shape of the second protruding portion (113a, 213a, 313a), and,
the second housing portion (113b, 213b, 313b) has a depressed shape in accordance with an outer shape of the first protruding portion (112a, 212a, 312a).

3. The stent (100) according to Claim 2, wherein in a state of being connected by the biodegradable material (121), the first connection section (112, 212, 312) and the second connection section (113, 213, 313) are disposed at positions where the first protruding portion (112a, 212a, 312a) is housed in the second housing portion (113b, 213b, 313b) and the first protruding portion (112a, 212a, 312a) and the second housing portion (113b, 213b, 313b) overlap with each other along a circumferential direction (D2) of the cylindrical shape.

4. The stent (100) according to any one of Claims 1 to 3, wherein each of the first connection section (112, 212, 312) and the second connection section (113, 213, 313) includes a holding portion (112c, 113c) formed so as to penetrate or be depressed from a surface of the strut (110) for holding the biodegradable material (121) in a thickness direction (D3), wherein
the holding portion (112c) of the first connection section (112, 212, 312) is disposed at a position overlapping the second connection section (113, 213, 313) along the axial direction (D1) of the cylindrical shape, and
the holding portion (113c) of the second connection section (113, 213, 313) is disposed at a position overlapping with the first connecting section (112, 212, 312) along the axial direction (D1) of the cylindrical shape.

5. The stent (100) according to any one of Claims 1 to 4, comprising:
a covering member (122) including a drug, which is capable of preventing proliferation of a neointima on a surface of the stent (100).

6. The stent (100) according to any one of Claims 1 to 5, wherein the biodegradable material (121) is selected from the following:
polylactic acid, polyglycolic acid, lactic acid-glycolic acid copolymer, polycaprolactone, lactic acid-caprolactone copolymer, glycolic acid-caprolactone copolymer, poly-γ-glutamic acid, collagen, and/or magnesium and zinc.

## Patentansprüche

1. Stent (100) umfassend:
lineare Streben (110), die einen Außenumfang einer zylindrischen Form mit Lücken dazwischen definieren; und
Verbindungsabschnitte (120, 130, 220, 230), die die Streben (110) mit den Lücken verbinden, wobei die Streben (110) eine Vielzahl von spiralförmigen Abschnitten (111) definieren, die zu einer Spirale um eine Achse geformt sind, die sich entlang einer axialen Richtung (D1) der zylindrischen Form erstreckt, und wobei der Verbindungsabschnitt (120, 130, 220, 320) ein erstes Verbindungsstück (112, 212, 312) und ein zweites Verbindungsstück (113, 213, 313) umfasst, die einstückig mit einem aneinandergrenzenden ersten spiralförmigen Abschnitt (111) und zweiten spiralförmigen Abschnitt (111) ausgebildet sind, und sich an Positionen befinden, die sich in einem einander gegenüberliegenden Zustand wenigstens teilweise entlang der axialen Richtung (D1) der zylindrischen Form überlappen, und ein biologisch abbaubares Material (121), das dazu ausgebildet ist, das erste Verbindungsstück (112, 212, 312) und das zweite Verbindungsstück (113, 213, 313) zu verbinden, indem es zwischen dem ersten Verbindungsstück (112, 212, 312) und dem zweiten Verbindungsstück (113, 213, 313) angeordnet ist, wobei
der erste spiralförmige Abschnitt (111) auf einer Anfangsstirnseite (A) der Vielzahl von spiralförmigen Abschnitten (111) in Bezug auf den zweiten spiralförmigen Abschnitt (111) angeordnet ist, und
wenigstens ein Teil des ersten Verbindungsstücks (112, 212, 312) auf der Anfangsstirnseite (A) der Vielzahl von spiralförmigen Abschnitten (111) in Bezug auf wenigstens einen Teil des zweiten Verbindungsstücks (113, 213, 313) angeordnet ist, wobei
das erste Verbindungsstück (112, 212, 312) Folgendes aufweist:
einen ersten vorspringenden Abschnitt (112a, 212a, 312a), der in Richtung zu dem zweiten Verbindungsstück (113, 213, 313) vorspringt, und
einen ersten Gehäuseabschnitt (112b, 212b, 312b), der sich bis zu dem ersten vorspringenden Abschnitt (112a, 212a, 312a) erstreckt, und
das zweite Verbindungsstück (113, 213, 313) Folgendes aufweist:
einen zweiten vorspringenden Abschnitt (113a, 213a, 313a), der in Richtung zu dem ersten Verbindungsstück (112, 212, 312) vorspringt, und
einen zweiten Gehäuseabschnitt (113b, 213b, 313b), der sich bis zu dem zweiten vorspringenden Abschnitt (113a, 213a, 313a) erstreckt,
**dadurch gekennzeichnet, dass**
der erste Gehäuseabschnitt (112b, 212b, 312b) des ersten Verbindungsstücks (112, 212, 312) auf der Anfangsstirnseite (A) der Vielzahl von spiralförmigen Abschnitten (111) in Bezug auf den zweiten Gehäuseabschnitt (113b, 213b, 313b) des zweiten Verbindungsstücks (113, 213, 313) angeordnet ist.

2. Stent (100) nach Anspruch 1, wobei der erste Gehäuseabschnitt (112b, 212b, 312b) eine vertiefte Form gemäß einer äußeren Form des zweiten vorspringenden Abschnitts (113a, 213a, 313a) hat, und der zweite Gehäuseabschnitt (113b, 213b, 313b) eine vertiefte Form gemäß einer äußeren Form des ersten vorspringenden Abschnitts (112a, 212a, 312a) hat.

3. Stent (100) nach Anspruch 2, wobei sich in einem Zustand der Verbindung durch das biologisch abbaubare Material (121) das erste Verbindungsstück (112, 212, 312) und das zweite Verbindungsstück (113, 213, 313) an Positionen befinden, wo der erste vorspringende Abschnitt (112a, 212a, 312a) in dem zweiten Gehäuseabschnitt (113b, 213b, 313b) aufgenommen ist und der erste vorspringende Abschnitt (112a, 212a, 312a) und der zweite Gehäuseabschnitt (113b, 213b, 313b) einander entlang einer Umfangsrichtung (D2) der zylindrischen Form überlappen.

4. Stent (100) nach einem der Ansprüche 1 bis 3, wobei das erste Verbindungsstück (112, 212, 312) und das zweite Verbindungsstück (113, 213, 313) jeweils einen Halteabschnitt (112c, 113c) aufweist, der so ausgebildet ist, dass er eine Oberfläche der Strebe (110) durchdringt oder bezüglich dieser vertieft ist, um das biologisch abbaubare Material (121) in einer Dickenrichtung (D3) zu halten, wobei
der Halteabschnitt (112c) des ersten Verbindungsstücks (112, 212, 312) sich an einer Position befindet, die das zweite Verbindungsstück (113, 213, 313) entlang der axialen Richtung (D1) der zylindrischen Form überlappt, und
der Halteabschnitt (113c) des zweiten Verbindungsstücks (113, 213, 313) sich an einer Position befindet, die sich mit dem ersten Verbindungsstück (112, 212, 313) entlang der axialen Richtung (D1) der zylindrischen Form überlappt.

5. Stent (100) nach einem der Ansprüche 1 bis 4, umfassend:
ein Abdeckelement (122) mit einem Arzneimittel, das in der Lage ist, die Proliferation einer Neointima auf einer Oberfläche des Stents (100) zu verhindern.

6. Stent (100) nach einem der Ansprüche 1 bis 5, wobei das biologisch abbaubare Material (121) aus Folgendem ausgewählt ist:
Polymilchsäure, Polyglycolsäure, Milchsäure-Glycolsäure-Copolymer, Polycaprolacton, Milchsäure-Caprolacton-Copolymer, Glycolsäure-Caprolacton-Copolymer, Poly-γ-glutaminsäure, Kollagen und/oder Magnesium und Zink.

## Revendications

1. Endoprothèse (100) comprenant :
des entretoises linéaires (110) qui définissent une périphérie extérieure d'une forme cylindrique avec des espaces entre elles ; et
des parties de liaison (120, 130, 220, 320) qui relient les entretoises (110) avec les espaces, où les entretoises (110) définissent une pluralité de parties hélicoïdales (111) formées en un forme d'hélice autour d'un axe s'étendant le long d'une direction axiale (D1) de la forme cylindrique, et où la partie de liaison (120, 130, 220, 320) comprend une première section de connexion (112, 212, 312) et une seconde section de connexion (113, 213, 313) formées de façon intégrante avec une première partie hélicoïdale (111) et une seconde partie hélicoïdale (111) adjacentes l'une à l'autre, et disposées en des positions se chevauchant au moins partiellement l'une l'autre le long de la direction axiale (D1) de la forme cylindrique dans un état où elles se font face l'une l'autre, et un matériau biodégradable (121) adapté pour relier la première section de connexion (112, 212, 312) et la seconde section de connexion (113, 213, 313) en étant intercalé entre la première section de connexion (112, 212, 312) et la seconde section de connexion (113, 213, 313), où
la première partie hélicoïdale (111) est disposée sur un côté d'extrémité de début (A) de la pluralité de parties hélicoïdales (111) par rapport à la seconde partie hélicoïdale (111), et
au moins une partie de la première section de connexion (112, 212, 312) est disposée sur le côté d'extrémité de début (A) de la pluralité de parties hélicoïdales (111) par rapport à au moins une partie de la seconde section de connexion (113, 213, 313), où
la première section de connexion (112, 212, 312) comprend :
une première partie saillante (112a, 212a, 312a) qui fait saillie vers la seconde section de connexion (113, 213, 313), et
une première partie de logement (112b, 212b, 312b) qui continue jusqu'à la première partie saillante (112a, 212a, 312a) et,
la seconde section de connexion (113, 213, 313) comprend :
une seconde partie saillante (113 a, 213a, 313a) qui fait saillie vers la première section de connexion (112, 212, 312), et
une seconde partie de logement (113b, 213b, 313b) qui continue jusqu'à la seconde partie saillante (113a, 213a, 313a),
**caractérisée en ce que**
la première partie de logement (112b, 212b, 312b) de la première section de connexion (112, 212, 312) est disposée sur le côté d'extrémité de début (A) de la pluralité de parties hélicoïdales (111) par rapport à la seconde partie de logement (113b, 213b, 313b) de la seconde section de connexion (113, 213, 313).

2. Endoprothèse (100) selon la revendication 1, dans laquelle la première partie de logement (112b, 212b, 312b) a une forme abaissée en fonction d'une forme extérieure de la seconde partie saillante (113a, 213a, 313a), et,
la seconde partie de logement (113b, 213b, 313b) a une forme abaissée en fonction d'une forme extérieure de la première partie saillante (112a, 212a, 312a).

3. Endoprothèse (100) selon la revendication 2, dans laquelle dans un état de liaison par le matériau biodégradable (121), la première section de connexion (112, 212, 312) et la seconde section de connexion (113, 213, 313) sont disposées en des positions où la première partie saillante (112a, 212a, 312a) est logée dans la seconde partie de logement (113b, 213b, 313b) et la première partie saillante (112a, 212a, 312a) et la seconde partie de logement (113b, 213b, 313b) se chevauchent l'une l'autre le long d'une direction circonférentielle (D2) de la forme cylindrique.

4. Endoprothèse (100) selon l'une quelconque des revendications 1 à 3, dans laquelle chacune de la première section de connexion (112, 212, 312) et de la seconde section de connexion (113, 213, 313) comprend une partie de support (112c, 113c) formée de manière à pénétrer ou à être abaissée depuis une surface de l'entretoise (110) pour supporter le matériau biodégradable (121) dans la direction de l'épaisseur (D3), où
la partie de support (112c) de la première section de connexion (112, 212, 312) est disposée en une position chevauchant la seconde section de connexion (113, 213, 313) le long de la direction axiale (D1) de la forme cylindrique, et
la partie de support (113c) de la seconde section de connexion (113, 213, 313) est disposée en une position chevauchant la première section de connexion (112, 212, 312) le long de la direction axiale (D1) de la forme cylindrique.

5. Endoprothèse (100) selon l'une quelconque des revendications 1 à 4, comprenant :
un élément de couverture (122) comprenant un médicament qui est capable d'empêcher la prolifération d'une néo-intima sur une surface de l'endoprothèse (100).

6. Endoprothèse (100) selon l'une quelconque des revendications 1 à 5, dans laquelle le matériau biodégradable (121) est choisi parmi les matériaux suivants :
acide polylactique, acide polyglycolique, copolymère d'acide lactique-acide glycolique, polycaprolactone, copolymère d'acide lactique-caprolactone, copolymère d'acide glycolique-caprolactone, acide poly-γ-glutamique, collagène, et/ou magnésium et zinc.
